# EUROPEAN PATENT APPLICATION

(11) **EP 2 623 490 A1**
(43) Date of publication of application: **07.08.2013**
(21) Application number: 12305130.2
(22) Date of filing: 03.02.2012
(51) Int. Cl.: C07C 217/84, A61P 27/02, A61K 31/196

(54) **2-(3-aminophenoxy)-acetic acid derivatives as EP2 receptor agonists for use in treatment of glaucoma**

(71) Applicant: Fovea Pharmaceuticals, 75012 Paris (FR)
(72) Inventor: Feutrill,John, Victoria 3084 (AU); Leriche,Caroline, 75012 Paris (FR); Middlemiss,David, Hertfodshire CM23 3NF (GB)
(74) Representative: Rauline, Mathilde

(57) **Abstract**

Aniline derivatives, their preparation and their therapeutic application as EP2 agonists in, for example, glaucoma. 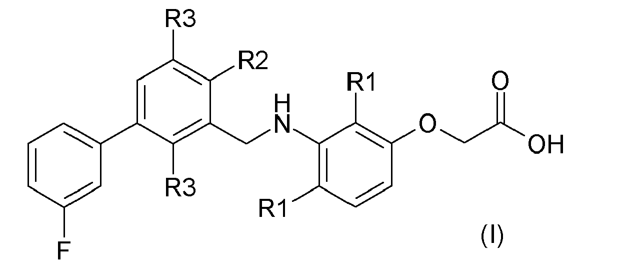 R1 represents, independently from each other, H, a (C₁-C₆) alkyl or an halogen,
R2 represents H, an halogen, or a (C₁-C₆) alkyl,
R3 represents, independently from each other, H, an halogen, -OH, -O-(C₁-C₆) alkyl or a -(C₁-C₆) alkyl.

## Description

Prostanoids comprise prostaglandins (PGs) and thromboxanes (Txs) and their receptors fall into five different classes (DP, EP, FP, IP and TP) based on their sensitivity to the five naturally occurring prostanoids, PGD2, PGE2, PGF2a, PGI2 and TxA2.

EP receptors (for which the endogenous ligand is PGE2) have been subdivided into four types termed EP1, EP2, EP3 and EP4. These four types of EP receptors have been cloned and are distinct at both a molecular and pharmacological level. EP2 agonists have been shown to be effective in the treatment of a number of conditions, including (but not limited to) dysmenorrhoea, pre-term labour, glaucoma, ocular hypertension, immune disorders, osteoporosis, asthma, allergy, bone disease, fracture repair, male sexual dysfunction, female sexual dysfunction, periodontal disease, gastric ulcer, and renal disease.

EP2 agonists have also been described in the treatment of inflammatory and immune disorders such as psoriasis, dermatitis, rheumatoid arthritis, multiple sclerosis, scleroderma, transplant rejection, allergy, systemic lupus erythematosus, vasculitis, type 1 diabetes mellitus, and inflammatory lung diseases such as chronic obstructive pulmonary disease, asthma, acute respiratory distress syndrome and cystic fibrosis.

In addition, EP2 agonists have also been described in the treatment of fibrosis, including, but not limited to scleroderma and systemic sclerosis, post-operative fibrosis following trabulectomy, liver repair and regeneration following cirrhosis, hepatitis, toxicity, cancer or renal fibrosis. EP2 agonists can also be used in the prevention of fibroblast to myofibroblast conversion to treat asthma and other fibrotic lung diseases. EP2 agonists may also be used to maintain ductus arteriosus patency in infants with congenital heart disease.

Glaucoma is a disease of the eye. It is a multifactorial, progressive optic neuropathy with a characteristic loss of retinal ganglion cells that form the optic nerve.

It is the second leading cause of blindness in the world. Prevalence model estimate that 8.4 millions individuals suffered from glaucoma-induced bilateral blindness in 2010, rising to 11.1 million in 2020. Glaucoma is asymptomatic in the early stages, that is why 50% of patients affected are undiagnosed. It then results in peripheral visual field loss and if untreated, can lead to irreversible blindness.

Primary glaucoma is a congenital glaucoma and secondary glaucoma results from pre-existing ocular diseases such as uveitis, intraocular tumor or enlarged cataract. Primary Chronic Open-angle glaucoma is generally associated with increased pressure in the eye caused by trabecular blockage. Not all people with primary open-angle glaucoma show an elevated intra-ocular pressure, but decreasing the eye pressure has been shown to stop progression even in these cases. Primary open-angle glaucoma is characterized by progressive visual field loss and optic nerves changes.

In Primary Chronic or acute Closed-angle glaucoma, the iridocorneal angle is completely closed because of forward displacement of the final roll and root of the iris against the cornea. This can lead to acute crises characterized by sudden ocular pain, seeing halos around lights, red eye, very high intraocular pressure, nausea and vomiting, and sudden decreased vision. Acute angle closure is an ocular emergency.

The diagnosis of glaucoma is generally made by intra-ocular pressure measurements, visual field tests and by looking for changes in the optic nerve.

The different treatments of glaucoma aim to reduce intra-ocular pressure.

Several treatments are currently available: for example, beta-adrenergic antogonists, carbonic anhydrase inhibitors or topical prodrugs of prostaglandins, like latanaprost, travoprost and bimatoprost.

Among them, the marketed topical prodrugs of protaglandins increase uveoscleral outflow and increase trabecular outflow facility. When applied in vivo, they release the active drug which is an agonist of the prostaglandin receptors F (FP agonists).

Therefore, there is a need for treatments that reduce intraocular pressure in order to treat and/or prevent ocular diseases involving EP2 receptors, such as glaucoma, for example.

The invention relates to EP2 agonists and their prodrugs, to their preparation and to their therapeutic application.

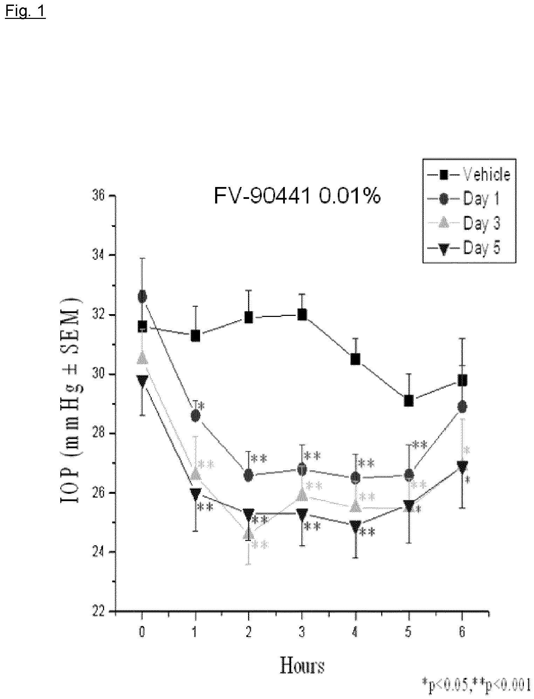

Fig. 1 shows the evolution of intraocular pressure (IOP) in monkeys suffering from glaucoma.

The present invention provides compounds of formula (I): 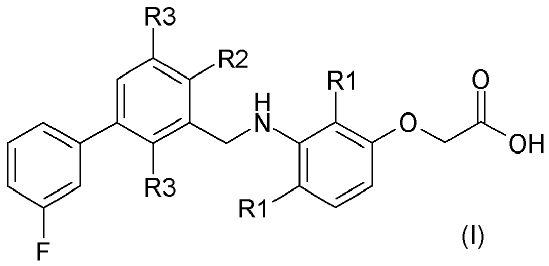 or one of its prodrugs, in which:
R1 represents, independently from each other, H, a (C₁-C₆) alkyl or an halogen,
R2 represents H, an halogen, or a (C₁-C₆)alkyl,
R3 represents, independently from each other, H, an halogen, -OH, -O-(C₁-C₆) alkyl or a (C₁-C₆) alkyl.

For example, the prodrugs of compounds of formula (I) can be compounds of formula (II): 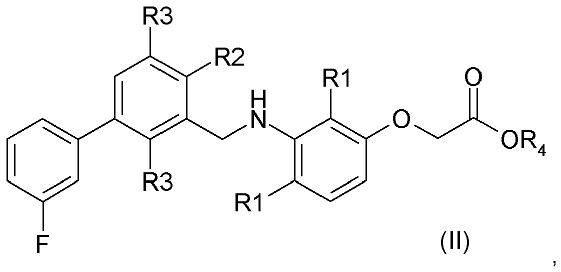 in which R1, R2 and R3 are as mentioned above, and R4 is (C₁-C₆) alkyl.

In the context of the present invention, the following terms have to be understood as:
- an halogen atom: a fluorine, a chlorine, a bromine or an iodine;
- a (C₁-C₆)alkyl : a linear or branched saturated hydrocarbon group. Examples include the groups methyl, ethyl, propyl, isopropyl, butyl, isobutyl, tert-butyl, pentyl, etc.;
- a prodrug: a compound that, on administration, must undergo chemical conversion by metabolic processes before becoming an active pharmacological agent. It is a compound administered in an inactive or significantly less active form than after its bioactivation. Once administered, the prodrug is metabolised in vivo into a therapeutically active compound (drug). This process is termed bioactivation. This bioactivation takes place in one or more steps, i.e. by providing one or more metabolites. A prodrug is usually not a therapeutically active compound itself and will usually not elicit in vitro the biological response of the corresponding therapeutically active compound after bioactivation.

According to the present invention bioactivation takes place particularly in the cornea. This can be tested ex-vivo using isolated rabbit cornea with Ussing chambers. For example, a prodrug is a compound in which a moiety is protected by a (C₁-C₆)alkyl, an ethyl-morpholine, -O-CH(CH₃)-OCOCH₃, -CH₂-CH₂-OH, -CH-(CH₂OH)₂, -CH₂-CH-(CH₂OH)₂, or -CH₂-C-(CH₂OH)₃. In particular, the prodrug can form an ester on the carboxylic moiety of the drug (compound (I), for example).

Among the compounds of formula (I) that are subject matter of the invention, mention may be made in particular of the following compounds:
N° 1: 2-[3-[[2-fluoro-5-(3-fluorophenyl)phenyl]methylamino]phenoxy]acetic acid
N° 2: 2-[2,4-difluoro-3-[[2-fluoro-5-(3-fluorophenyl)phenyl]methylamino]phenoxy] acetic acid
N° 6: 2-[2,4-difluoro-3-[[5-(3-fluorophenyl)-2-methyl-phenyl]methylamino]phenoxy] acetic acid
N° 7: 2-[2,4-difluoro-3-[[2-fluoro-3-(3-fluorophenyl)-5-hydroxy-phenyl]methylamino] phenoxy]acetic acid
N° 8: 2-[2,4-difluoro-3-[[3-(3-fluorophenyl)phenyl]methylamino]phenoxy]acetic acid
N° 9: 2-[2,4-difluoro-3-[[2-fluoro-3-(3-fluorophenyl)-5-methyl-phenyl]methylamino] phenoxy]acetic acid
N° 10: 2-[2,4-difluoro-3-[[3-(3-fluorophenyl)-5-methyl-phenyl]methylamino]phenoxy] acetic acid
N° 11: 2-[2,4-difluoro-3-[[2-fluoro-5-(3-fluorophenyl)-3-methyl-phenyl]methylamino] phenoxy]acetic acid
N° 12: 2-[2-fluoro-3-[[2-fluoro-5-(3-fluorophenyl)phenyl]methylamino]-4-methyl-phenoxy] acetic acid
N° 13: 2-[3-[[2-fluoro-5-(3-fluorophenyl)phenyl]methylamino]-2,4-dimethyl-phenoxy]acetic acid
N° 14: 2-[4-chloro-2-fluoro-3-[[2-fluoro-5-(3-fluorophenyl)phenyl]methylamino]phenoxy] acetic acid
N° 15: 2-[3-[[2-chloro-5-(3-fluorophenyl)phenyl]methylamino]-2,4-difluoro-phenoxy]acetic acid
N° 16: 2-[4-fluoro-3-[[2-fluoro-5-(3-fluorophenyl)phenyl]methylamino]-2-methyl-phenoxy] acetic acid
N° 17: 2-[2,4-difluoro-3-[[3-fluoro-5-(3-fluorophenyl)phenyl]methylamino]phenoxy]acetic acid
N° 18: 2-[2-chloro-4-fluoro-3-[[2-fluoro-5-(3-fluorophenyl)phenyl]methylamino]phenoxy] acetic acid
N° 19: 2-[2,4-difluoro-3-[[3-(3-fluorophenyl)-5-hydroxy-phenyl]methylamino]phenoxy] acetic acid
N° 20: 2-[2,4-difluoro-3-[[3-(3-fluorophenyl)-5-methoxy-phenyl]methylamino]phenoxy] acetic acid
N° 21: 2-[2,4-dichloro-3-[[2-fluoro-5-(3-fluorophenyl)phenyl]methylamino]phenoxy]acetic acid

Among the prodrugs of compounds of formula (I) that are subject matter of the invention, mention may be made in particular of the following compounds:
N° 3: methyl 2-[2,4-difluoro-3-[[2-fluoro-5-(3-fluorophenyl)phenyl]methylamino] phenoxy] acetate
N° 4: ethyl 2-[2,4-difluoro-3-[[2-fluoro-5-(3-fluorophenyl)phenyl]methylamino] phenoxy] acetate
N° 5: isopropyl-[2,4-difluoro-3-[[2-fluoro-5-(3-fluorophenyl)phenyl]methylamino] phenoxy]acetate

In one aspect of the invention, compounds of formula (I) according to the invention do are not the following molecules:

| Example Nb. | Formula | Chemical name |
|---|---|---|
| N° A | 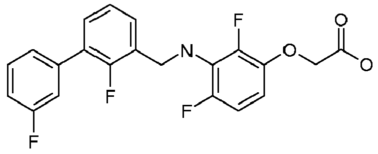 | 2-[2, 4-d ifl uoro-3-[[2-fluoro-3-(3-fl uor ophenyl)phenyl]methylamino]pheno xy]acetic acid |
| N° B | 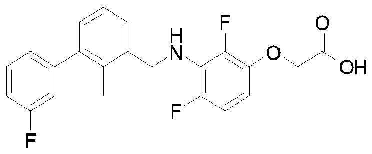 | 2-[2,4-difluoro-3-[[3-(3-fluorophenyl) -2-methyl-phenyl]methylamino]phen oxy]acetic acid |
| N° C | 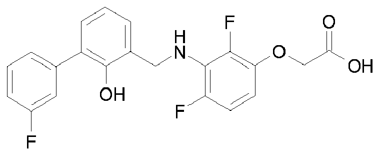 | 2-[2,4-difluoro-3-[[3-(3-fluorophenyl) -2-hydroxy-phenyl]methylamino]phe noxy]acetic acid |
| N° D | 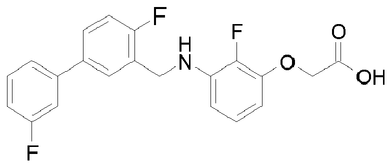 | 2-[2-fluoro-3-[[2-fluoro-5-(3-fluoroph enyl)phenyl]methylamino]phenoxy]a cetic acid |
| N° E | 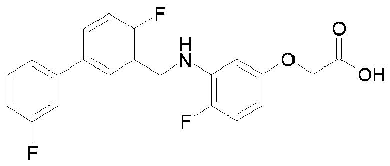 | 2-[4-fluoro-3-[[2-fluoro-5-(3-fluoroph enyl)phenyl]methylamino]phenoxy]a cetic acid |

In another aspect of the present invention, compounds of formula (I) include compounds with R2 and R3 as mentioned above, and R1 represents, independently from each other, a (C₁-C₆) alkyl or an halogen.

In accordance with the invention the compounds of general formula (I) can be prepared by the following process.

The bi-aryl moiety can be prepared according to scheme 1.

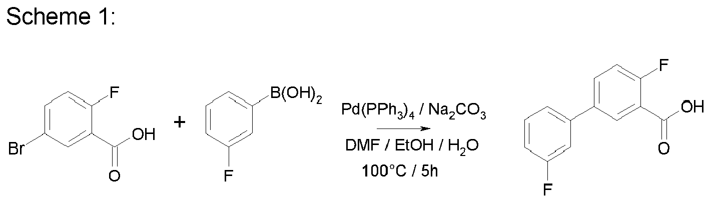

From the product obtained in Scheme 1, an example of the process to prepare the compound of formula (I) according to the invention is described in Scheme 2.

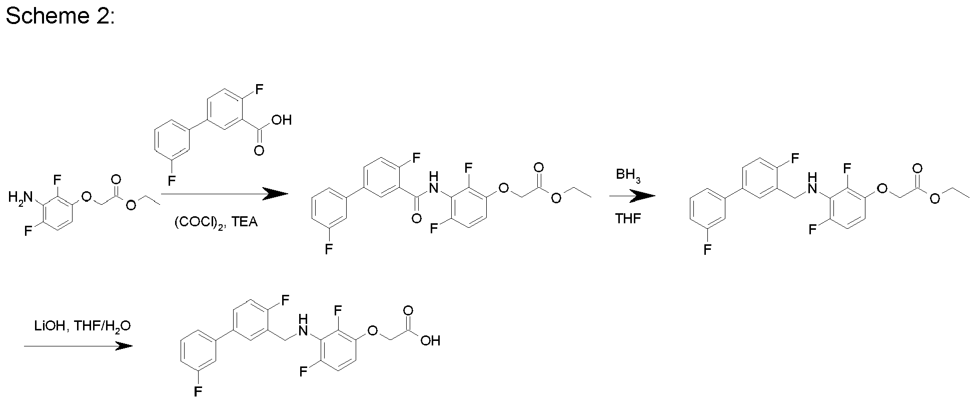

Prodrugs can also be synthesized according to scheme 3.

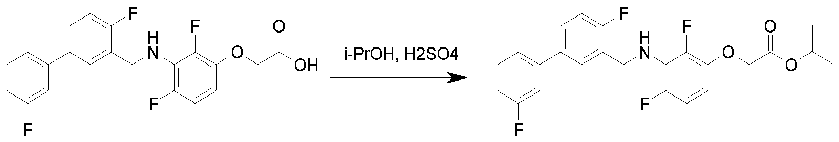

In schemes 1 to 3, the starting compounds and the reactants, when the way in which they are prepared is not described, are available commercially or are described in the literature, or else may be prepared by methods which are described therein or which are known to a person skilled in the art.

The examples which follow describe the preparation of certain compounds in accordance with the invention. These examples are not limitative, and merely illustrate the present invention. The numbers of the compounds exemplified match those given in the table hereinafter, which illustrates the chemical structures and physical properties of some compounds according to the invention.

The table below illustrates the chemical structures and the physical properties of some examples of compounds according to the invention.

| Example Nb. | Formula | Chemical name |
|---|---|---|
| N° 1 | 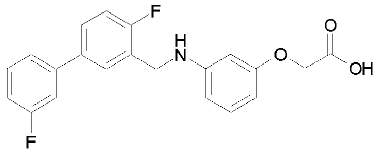 | 2-[3-[[2-fluoro-5-(3-fluorophen yl)phenyl]methylamino]pheno xy]acetic acid |
| N° 2 | 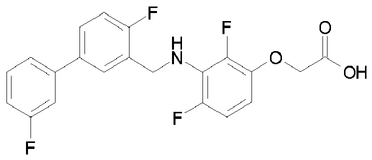 | 2-[2,4-difluoro-3-[[2-fluoro-5-( 3-fluorophenyl)phenyl]methyl amino]phenoxy]acetic acid |
| N° 3 | 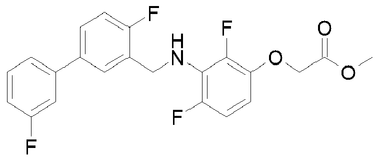 | methyl 2-[2,4-difluoro-3-[[2-fluoro-5-( 3-fluorophenyl)phenyl]methyl amino]phenoxy]acetate |
| N° 4 | 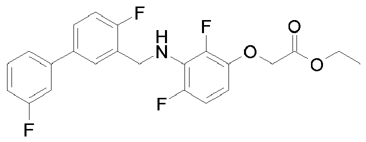 | ethyl 2-[2,4-difluoro-3-[[2-fluoro-5-( 3-fluorophenyl)phenyl]methyl amino]phenoxy]acetate |
| N°5 | 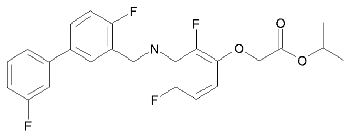 | isopropyl 2-[2,4-difluoro-3-[[2-fluoro-5-( 3-fl uorophenyl) phenyl] methyl amino]phenoxy]acetate |
| N° 6 | 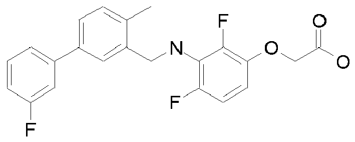 | 2-[2,4-difluoro-3-[[5-(3-fluorop henyl)-2-methyl-phenyl]methy lamino]phenoxy]acetic acid |
| N° 7 | 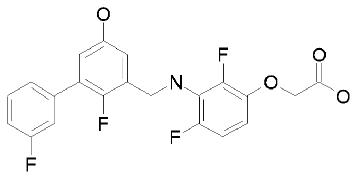 | 2-[2,4-difluoro-3-[[2-fluoro-3-( 3-fluorophenyl)-5-hydroxy-ph enyl]methylamino]phenoxy]ac etic acid |
| N° 8 | 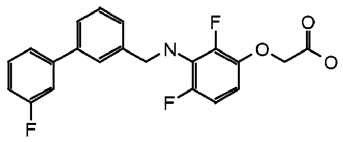 | 2-[2,4-difluoro-3-[[3-(3-fluorop henyl)phenyl]methylamino]ph enoxy]acetic acid |
| N° 9 | 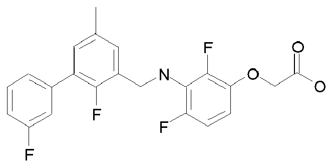 | 2-[2,4-difluoro-3-[[2-fluoro-3-( 3-fluorophenyl)-5-methyl-phe nyl]methylamino]phenoxy]ace tic acid |
| N° 10 | 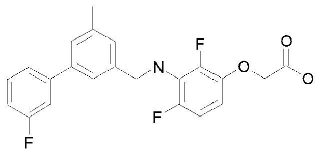 | 2-[2,4-difluoro-3-[[3-(3-fluorop henyl)-5-methyl-phenyl]methy lamino]phenoxy]acetic acid |
| N° 11 | 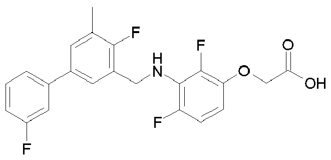 | 2-[2,4-difluoro-3-[[2-fluoro-5-( 3-fluorophenyl)-3-methyl-phe nyl]methylamino]phenoxy]ace tic acid |
| N° 12 | 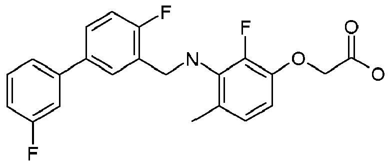 | 2-[2-fluoro-3-[[2-fluoro-5-(3-fl uorophenyl)phenyl]methylami no]-4-methyl-phenoxy]acetic acid |
| N° 13 | 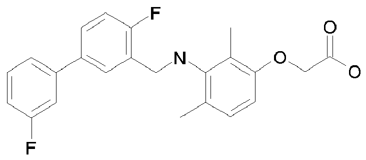 | 2-[3-[[2-fluoro-5-(3-fluorophen yl)phenyl]methylamino]-2,4-di methyl-phenoxy]acetic acid |
| N° 14 | 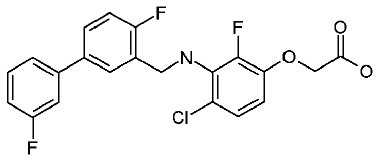 | 2-[4-chloro-2-fluoro-3-[[2-fluor o-5-(3-fluorophenyl)phenyl]m ethylamino]phenoxy]acetic acid |
| N° 15 | 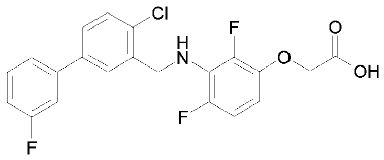 | 2-[3-[[2-chloro-5-(3-fluorophe nyl)phenyl]methylamino]-2,4-difluoro-phenoxy]acetic acid |
| N° 16 | 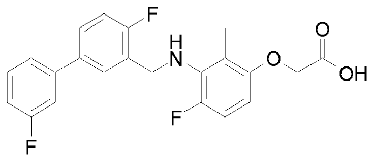 | 2-[4-fluoro-3-[[2-fluoro-5-(3-fl uorophenyl)phenyl]methylami no]-2-methyl-phenoxy]acetic acid |
| N° 17 | 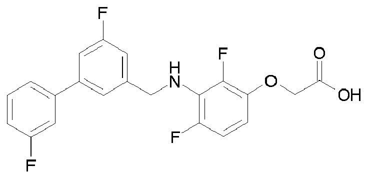 | 2-[2,4-difluoro-3-[[3-fluoro-5-( 3-fluorophenyl)phenyl]methyl amino]phenoxy]acetic acid |
| N° 18 | 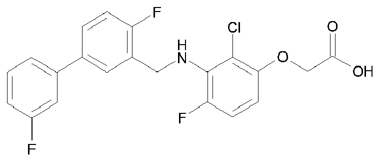 | 2-[2-chloro-4-fluoro-3-[[2-fluor o-5-(3-fluorophenyl)phenyl]m ethylamino]phenoxy]acetic acid |
| N° 19 | 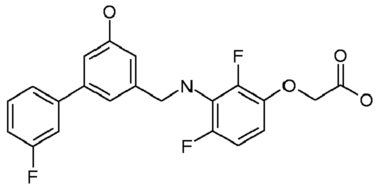 | 2-[2,4-difluoro-3-[[3-(3-fluorop henyl)-5-hydroxy-phenyl]met hylamino]phenoxy]acetic acid |
| N° 20 | 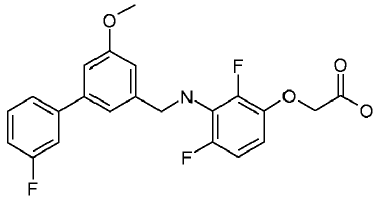 | 2-[2,4-difluoro-3-[[3-(3-fluorop henyl)-5-methoxy-phenyl]met hylamino]phenoxy]acetic acid |
| N° 21 | 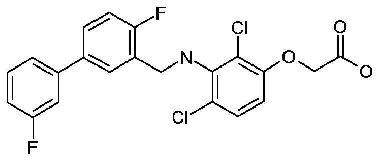 | 2-[2,4-dichloro-3-[[2-fluoro-5-( 3-fluorophenyl)phenyl]methyl amino]phenoxy]acetic acid |

(IUPAC chemical name have been attributed by the SymyxDraw program, version 3.2)

The compounds according to the table were subjected to pharmacological tests for determining their effect on EP2 receptors.

The prodrugs according to the table were subjected to corneal permeability and hydrolysis studies.

### EXAMPLES

The following non-limiting preparations and Examples illustrate the preparation of the compounds of the invention. ¹H Nuclear Magnetic Resonance (NMR) spectra were in all cases consistent with the proposed structures. We used a Fourier 300 MHz Bruker. Characteristic chemical shifts δ are given in parts-per-million downfield from tetramethylsilane using conventional abbreviations for designation of major peaks: e.g. s, singlet; d, doublet; t, triplet; q, quartet; m, multiplet; bb, broad band. The mass spectra (m/z) (Agilent MSD) were recorded using electrospray ionisation (ESI). The following abbreviations have been used for common solvents:
d6-DMSO, deuterodimethylsulphoxide,
EtOH, ethanol,
DMF, dimethylformamide,
EtOAc, ethyl acetate,
DCM, dichloromethane,
TEA, triethylamine.

Where thin layer chromatography (TLC) has been used it refers to silica gel 60 F254 plates, R_{f} is the distance travelled by a compound divided by the distance travelled by the solvent front on a TLC plate.

### Example 1: Preparation of isopropyl 2-[2,4-difluoro-3-[[2-fluoro-5-(3-fluorophenyl)phenyl] ethylamino]phenoxy]acetate

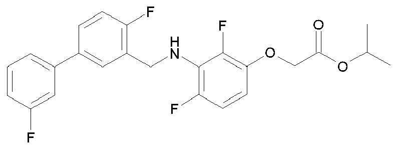

### A. Preparation of 2-fluoro-5-(3-fluorophenyl)benzoic acid

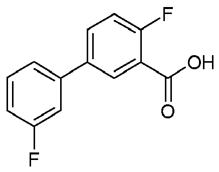

To a mixture of 5-bromo-2-fluorobenzoic acid (3g, 13.2 mmol) and 3-fluorophenyl boronic acid (2.1 g, 15.1 mmol) in water (15 ml), EtOH (15 ml) and DMF (60 ml) was added Na₂CO₃ (5.81 g, 54.8 mmol) and Pd(PPh₃)₄ (1.58 g, 1.37 mmol). The reaction mixture was heated to 100°C under nitrogen during 4h, then cooled to room temperature. The resulting mixture was poured into water and extracted with ethyl acetate. The organic layers were combined, dried over Na₂SO₄, filtered and concentrated to give 3g (12.8 mmol, 97%) of a white powder.

### B. Preparation of ethyl 2-(3-amino-2,4-difluoro-phenoxy)acetate

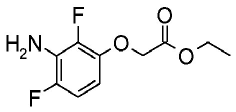

To a stirred solution of 3-amino-2,4-difluoro-phenol (713 mg, 4.91 mmol, 1.0 eq) in DMF (30 mL) was added Na₂CO₃ (1.56g, 14.75mmol, 3.0 eq). The resulting mixture was stirred for 40 min at RT. Ethyl bromoacetate (1 g, 5.90 mmol 1.2eq) was added and the reaction stirred overnight at RT. The reaction was diluted with water then extracted with EtOAc (60 mL x 3). The combined organic layers were washed with brine (50 mL x 2), dried over (Na₂SO₄) and concentrated under reduced pressure. The residue was purified by chromatography (Petroleum ether :EtOAc =25:1) to give ethyl 2-(3-amino-2,4-difluorophenoxy)acetate (850mg, 70.8%) as an oil.

### C. Preparation of 2-[2,4-difluoro-3-[[2-fluoro-5-(3-fluorophenyl)phenyl] methylamino]phenoxy] acetic acid

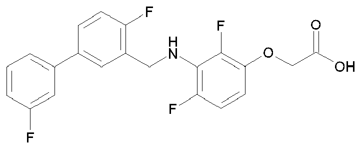

2-fluoro-5-(3-fluorophenyl)benzoic acid (330 mg, 1.4 mmol, 1.1 eq) was suspended in SOCl₂ (10 mL) and heated to reflux for 3 hrs. The solution was then concentrated and the residue was dissolved in dry DCM (10 mL). Ethyl 2-(3-amino-2,4-difluoro-phenoxy) acetate (300 mg, 1.3 mmol, 1eq) and TEA (260 mg, 2.6 mmol, 2eq. ) were added and the mixture was stirred at room temperature for 3 hrs. TLC indicated the completion of the reaction. Water was added and extracted with CH₂Cl₂ (30 mL x 3). The combined organic layers were washed with brine (50 mL x 2), dried over (Na₂SO₄) and concentrated under reduced pressure. The residue was purified by chromatography (Hexane /ethyl acetate, 10/1 to 5/1, v/v) to give ethyl 2-[2,4-difluoro-3-[[2-fluoro-5-(3-fluorophenyl)benzoyl]amino]phenoxy] acetate (290 mg, 49.9%) as a white solid.

Ethyl 2-[2,4-difluoro-3-[[2-fluoro-5-(3-fluorophenyl)benzoyl]amino]phenoxy]acetate (250 mg, 0.56 mmol, 1.0 eq) was dissolved in 10 mL dry THF and cooled to 0°C. A solution of BH₃ in THF (1M, 2.8mL, 2.8mmol) was added dropwise. The solution was then heated at 60°C for 2hrs. TLC indicated the completion of the reaction. The reaction was quenched with methanol and concentrated. Purification by column chromatography (DCM/MeOH, 50/1 to 20/1, v/v) provided ethyl 2-[2,4-difluoro-3-[[2-fluoro-5-(3-fluorophenyl)phenyl]methylamino]phenoxy]acetate as a colorless oil (100 mg, 41.2%).

Ethyl 2-[2,4-difluoro-3-[[2-fluoro-5-(3-fluorophenyl)phenyl]methylamino]phenoxy] acetate (100 mg, 0.2 mmol, 1.0 eq) was dissolved in a mixture of THF and H₂O (15 mL/10 mL). LiOH.2H₂O (55mg, 0.8mmol) was added and the solution was heated at 60°C overnight. TLC indicated the completion of the reaction. The THF was removed from the reaction under reduced pressure and the water phase extracted with EtOAc (60 mL x 3). The aqueous layer was acidified with 1M HCl and extracted with EtOAc (60 mL x 3). The combined organic layers were washed with brine (50 mL x 2), dried over (Na2SO4) and concentrated under reduced pressure to give the target product as a white solid (80 mg, 98.8%)

### D. Preparation of isopropyl2-[2,4-difluoro-3-[[2-fluoro-5-(3-fluorophenyl)phenyl] methylamino] phenoxy]acetate

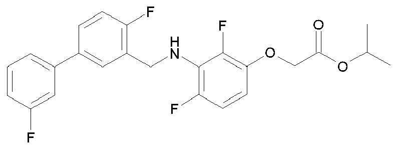

2-[2,4-difluoro-3-[[2-fluoro-5-(3-fluorophenyl)phenyl]methylamino]phenoxy]acetic acid (70 mg, 0.17 mmol, 1.0 eq) was dissolved in iso-propanol (5 mL). 0.05 mL of concentrated sulfuric acid was added. The solution was stirred at room temperature overnight. TLC indicated the consumption of starting material. The reaction was quenched by addition of aqueous NaHCO₃ and the aqueous layer extracted with ethyl acetate (30 mL x 3) the combined organic layers were dried over MgSO₄ and concentrated to give crude product. Recrystalization from petroleum ether and DCM then provided target product (52 mg, 68.4%) as a white solid.

**Human prostanoid EP₄ receptor binding assay**

| **Compound** | **Structure** | **MS ¹H NMR (300MHz, d6-DMSO)** |
|---|---|---|
| N° 1 | 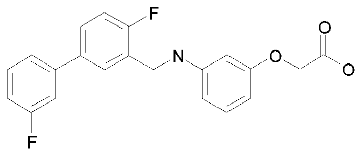 | M+1=370.1 NMR: δ 12.89 (bb, 1H, COOH); 10.77 (s, 1H); 7.74(dd, 1H); 7.63 (m, 1H); 7.51-7.42 (m, 3H); 7.29 (t,1H); 7.18 (m, 1H); 6.95 (t, 1H); 6.26 (dd, 1H); 6.17 (s, 1H); 6.08 (dd, 1H); 4.52 (s, 2H); 4.32 (d, 2H) |
| N° 2 | 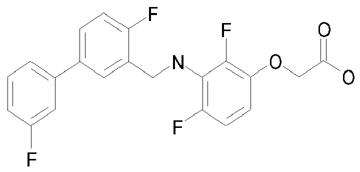 | M+1=406.0 NMR: δ 13.02 (bb, 1H, COOH); 7.74 (dd, 1H); 7.59 (m, 1H); 7.49 (m, 1H); 7.38 (m, 2H); 7.23 (t, 1H); 7.18 (td, 1H); 6.80 (td,1H); 6.32 (td, 1H); 5.84 (m, 1H); 4.63 (s, 2H); 4.52 (d, 2H) |
| N° 3 | 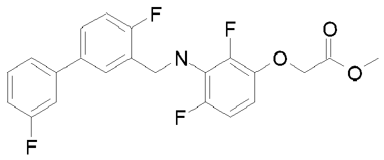 | M+1= 419.9 NMR: δ 7.73 (d, 1H); 7.59 (m, 1H); 7.48 (q, 1H); 7.41 (m, 2H); 7.23 (m, 2H); 6.80 (t, 1H); 6.35 (m, 1H); 5.88 (m, 1H); 4.76 (s, 2H); 4.52 (d, 2H); 3.65 (s, 3H) |
| N° 4 | 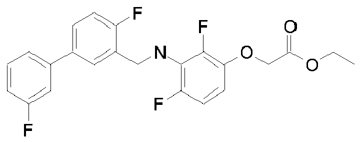 | M+1= 434.0 NMR: δ 7.73 (d, 1H); 7.59 (m, 1H); 7.48 (q, 1H); 7.41 (m, 2H); 7.23 (m, 2H); 6.80 (t, 1H); 6.35 (m, 1H); 5.88 (m, 1H); 4.74 (s, 2H); 4.52 (d, 2H); 4.11 (q, 2H); 1.15 (t, 3H) |
| N° 5 | 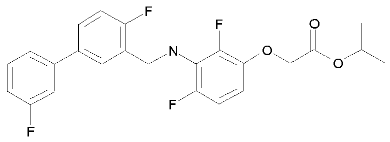 | M+1= 448.0 NMR: δ 7.73 (d, 1H); 7.59 (m, 1H); 7.48 (q, 1H); 7.41 (m, 2H); 7.23 (m, 2H); 6.80 (t, 1H); 6.32 (m, 1H); 5.88 (m, 1H); 4.93 (m, 1H); 4.70 (s, 2H); 4.52 (d, 2H); 1.14 (d, 6H) |
| N° 6 | 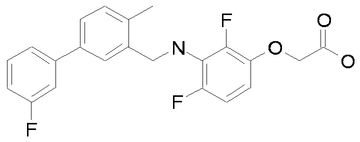 | M+1= 401.9 NMR: δ (13.03, bb, 1H, COOH); 7.59 (s, 1H); 7.51-7.43 (m, 2H); 7.39-7.33 (m, 2H); 7.24 (d, 1H); 7.15 (m, 1H); 6.80 (m, 1H); 6.31 (m, 1H); 5.73 (m, 1H); 4.63 (s, 2H); 4.46 (d, 2H); 2.32 (s, 3H) |
| N° 7 | 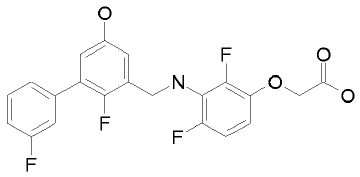 | M+1= 421.9 NMR: δ (13.08, bb, 1H, COOH); 9.44 (s, 1H); 7.50 (q, 1H); 7.39-7.20 (m, 3H); 6.89 (m, 1H); 6.79 (m, 1H); 6.68 (t, 1H); 6.19 (m, 1H); 5.72 (m, 1H); 4.68 (s, 2H); 4.47 (d, 2H) |
| N° 8 | 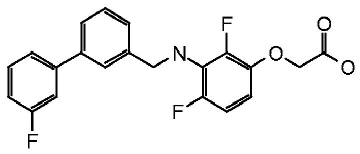 | M+1= 387.9 NMR: δ (13.03, bb, 1H, COOH); 7.65 (s, 1H); 7.54-7.42 (m, 4H); 7.39 (t, 1H); 7.31 (d, 1H); 7.19 (m, 1H); 6.77 (m, 1H); 6.27 (m, 1H); 5.98 (m, 1H); 4.61 (s, 2H); 4.46 (d, 2H) |
| N° 9 | 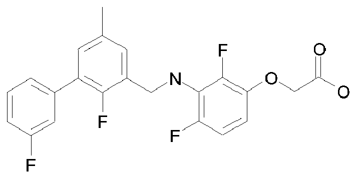 | M+1= 419.9 NMR: δ (13.05, bb, 1H, COOH); 7.50 (q, 1H); 7.36-7.20 (m, 5H); 6.79 (t, 1H); 6.30 (m, 1H); 5.81 (m, 1H); 4.63 (s, 2H); 4.49 (d, 2H); 2.28 (s, 3H) |
| N° 10 | 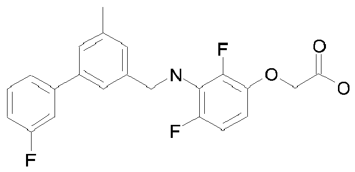 | M+1= 401.9 NMR: δ (13.08, bb, 1H, COOH); 7.52-7.35 (m, 4H); 7.35 (s, 1H); 7.17 (t, 1H); 7.13 (s, 1H); 6.77 (t, 1H); 6.27 (m, 1H); 5.93 (m, 1H); 4.61 (s, 2H); 4.42 (d, 2H); 2.23 (s, 3H) |
| N° 11 | 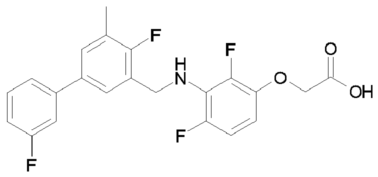 | M+1= 419.9 1H NMR (300MHz, DMSO-d6) d = 13.04 (br. s., 1H), 7.62-7.42 (m, 3H), 7.40 - 7.31 (m, 2H), 7.24 - 7.10 (m, 1H), 6.80 (ddd, J=2.1, 9.4, 11.7 Hz, 1H), 6.32 (dt, J=4.5, 9.1 Hz, 1H), 5.84 (br. s., 1H), 4.63 (s, 2H), 4.51 (d, J=7.1 Hz, 2H), 2.27 (d, J=1.6 Hz, 3H) |
| N° 12 | 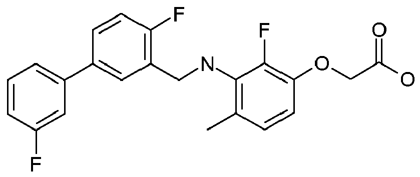 | M+1= 402.0 1H NMR (300MHz, DMSO-d6) d = 7.72 (dd, J=2.2, 7.2 Hz, 1H), 7.63 - 7.54 (m, 1H), 7.53-7.44 (m, 1H), 7.40 (s, 1H), 7.39 - 7.34 (m, 1H), 7.30-7.18 (m, 1H), 7.21 - 7.09 (m, 1H), 6.67 (d, J=8.0 Hz, 1H), 6.25 (t, J=8.2 Hz, 1H), 4.48 (s, 2H), 4.44 (s, 2H), 2.12 (s, 3H) |
| N° 13 | 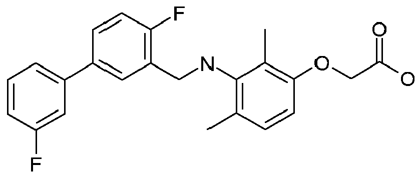 | M+1= 398.0 1H NMR (300MHz, DMSO-d6) d = 7.80 (dd, J=2.3, 7.1 Hz, 1H), 7.72 - 7.59 (m, 1H), 7.56-7.37 (m, 3H), 7.31 - 7.12 (m, 2H), 6.84 (d, J=8.3 Hz, 1H), 6.38 (d, J=8.3 Hz, 1H), 4.58 (s, 2H), 4.15 (s, 2H), 2.12 (d, J=2.4 Hz, 6H) |
| N° 14 | 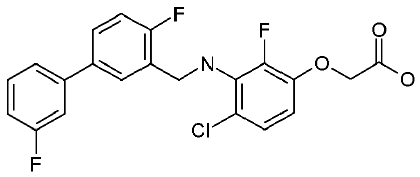 | M+1= 421.9 1H NMR (300MHz, DMSO-d6) d = 7.70 (dd, J=2.2, 7.2 Hz, 1 H), 7.64 - 7.55 (m, 1H), 7.53-7.42 (m, 1H), 7.42 - 7.32 (m, 2H), 7.30 - 7.19 (m, 1H), 7.23-7.12 (m, 1H), 7.03 (dd, J=2.0, 8.9 Hz, 1H), 6.41 (t, J=8.8 Hz, 1H), 5.67 (t, J=6.1 Hz, 1H), 4.65 (s, 2H), 4.60 (d, J=6.3 Hz, 2H) |
| N° 15 | 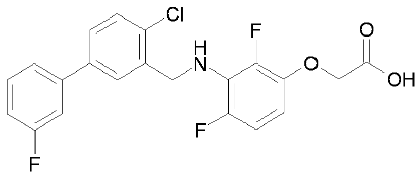 | 1H NMR (300MHz, DMSO-d6) d = 7.74 (d, J=2.0 Hz, 1H), 7.65 - 7.56 (m, 1H), 7.55-7.45 (m, 2H), 7.43 - 7.35 (m, 2H), 7.31 - 7.09 (m, 1H), 6.82 (ddd, J=2.1, 9.4, 11.7 Hz, 1H), 6.33 (dt, J=4.6, 9.1 Hz, 1H), 6.04 - 5.86 (m, 1H), 4.64 (s, 2H), 4.56 (d, J=6.8 Hz, 2H) |
| N° 16 | 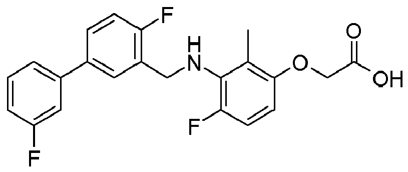 | 1H NMR (300MHz, DMSO-d6) d = 7.73 (dd, J=2.3, 7.2 Hz, 1H), 7.63 - 7.54 (m, 1H), 7.53-7.44 (m, 1H), 7.39 (d, J=7.7 Hz, 2H), 7.30 - 7.02 (m, 2H), 6.76 (dd, J=9.0, 12.3 Hz, 1H), 6.21 (dd, J=3.7, 9.0 Hz, 1H), 5.93 (m, 1H), 4.50 - 4.40 (m, 4H), 2.10 (s, 3H) |
| N° 17 | 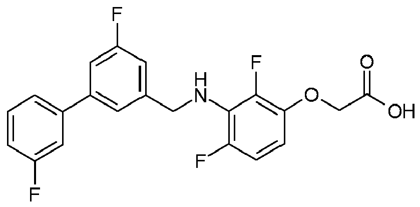 | 1H NMR (300MHz, DMSO-d6) d = 7.63 - 7.46 (m, 4H), 7.40 (d, J=10.1 Hz, 1H), 7.29 - 7.17 (m, 1H), 7.13 (d, J=9.7 Hz, 1H), 6.70 (ddd, J=2.1, 9.4, 11.7 Hz, 1H), 6.15 (dt, J=4.7, 9.2 Hz, 1H), 5.96 - 5.80 (m, 1H), 4.45 (d, J=7.3 Hz, 2H), 4.05 (s, 2H) |
| N° 18 | 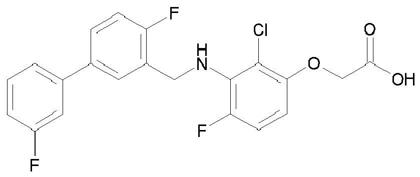 | 1H NMR (300MHz, DMSO-d6) d = 7.69 (dd, J=2.2, 7.2 Hz, 1H), 7.63 - 7.55 (m, 1H), 7.53-7.43 (m, 1H), 7.43 - 7.32 (m, 2H), 7.23 (dd, J=8.6, 9.8 Hz, 1H), 7.22 - 7.10 (m, 1H), 6.94 (dd, J=9.2, 12.6 Hz, 1H), 6.30 (dd, J=3.8, 9.2 Hz, 1H), 5.73 (t, J=7.1 Hz, 1 H), 4.85 - 4.43 (m, 4H) |
| N° 19 | 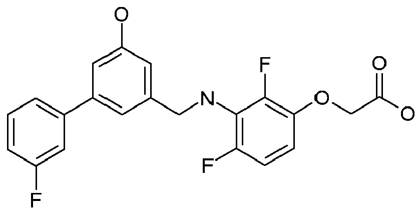 | M+1 = 404.0 1H NMR (300MHz, DMSO-d6) d = 7.59 - 7.42 (m, 1H), 7.41-7.27 (m, 2H), 7.25 - 7.09 (m, 1H), 7.05 (s, 1H), 6.86 (s, 1H), 6.82 - 6.75 (m, 1H), 6.74 (s, 1H), 6.27 (dt, J=4.5, 9.0 Hz, 1H), 5.92 (s, 1H), 4.62 (s, 2H), 4.38 (d, J=6.9 Hz, 2H) |
| N° 20 | 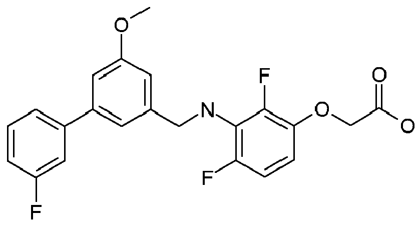 | M+1 = 417.9 1H NMR (300MHz, DMSO-d6) d = 7.61 - 7.36 (m, 3H), 7.36-7.14 (m, 2H), 7.05 (s, 1H), 6.89 (s, 1H), 6.85 - 6.64 (m, 1H), 6.28 (dt, J=4.6, 9.1 Hz, 1H), 5.96 (s, 1H), 4.62 (s, 2H), 4.42 (d, J=6.9 Hz, 2H), 3.78 (s, 3H) |
| N° 21 | 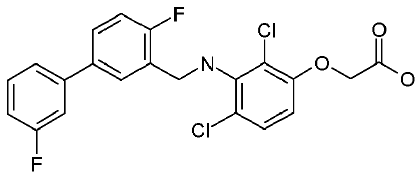 | M+1 = 437.9 1H NMR (300MHz, DMSO-d6) d = 7.96 - 7.73 (m, 1H), 7.59 (dt, J=2.4, 5.4 Hz, 1H), 7.54-7.45 (m, 1H), 7.44 - 7.33 (m, 2H), 7.31 - 7.00 (m, 3H), 6.49 (d, J=9.0 Hz, 1H), 5.52 - 5.18 (m, 1H), 4.62 (d, J=5.6 Hz, 2H), 4.58 (s, 2H) |

### Protocol

Cell membrane homogenate from Human embryonic kidney HEK-293(T) cells expressing recombinant human prostanoid EP₄ receptor was used to perform Prostanoid receptor radioligand binding assays.

The competition reaction was initiated by incubation of membrane protein homogenate (20 µg protein) for 120 min at 22°C with 0.5 nM [³H]PGE₂ ligand in the absence or presence of the test compound in a buffer containing 10 mM MES/KOH (pH 6.0), 10 mM MgCl₂ and 1 mM EDTA.

Non-specific binding was determined in the presence of 10 µM PGE₂ (the corresponding non-radioactive prostanoid). Affinity of the compound binding to hEP4 receptor was measured by displacement of the radiolabeled ligand in the presence of varying doses of tested compound.

Incubations were terminated by rapid filtration under vacuum through glass fiber filters (GF/B, Packard) presoaked with 0.3% PEI and washed several times with ice-cold 50 mM Tris-HCI using a 96-sample cell harvester (Unifilter, Packard).

The filters were dried and the residual radioactivity bound to the individual filters determined with addition of scintillation cocktail (Microscint 0, Packard) by a scintillation counter (Topcount, Packard).

The results were expressed as a percent inhibition of the control radioligand specific binding.

The standard reference compound PGE₂, was tested in at several concentrations to obtain a competition curve to determined IC_{50.}

### Analysis and expression of results

The specific ligand binding to the receptors is defined as the difference between the total binding and the nonspecific binding determined in the presence of an excess of unlabelled ligand. The results are expressed as a percent of control specific binding ((measured specific binding/control specific binding) x 100) obtained in the presence of the test compounds. The IC50 values (concentration causing a half-maximal inhibition of control specific binding) and Hill coefficients (nH) were determined by non-linear regression analysis of the competition curves generated with mean replicate values using Hill equation curve fitting (Y = D + [(A - D)/(1 + (C/C50)nH)], where Y = specific binding, D = minimum specific binding, A = maximum specific binding, C = compound concentration, C50 = IC50, and nH = slope factor). This analysis was performed using a software developed at Cerep (Hill software) and validated by comparison with data generated by the commercial software SigmaPlot® 4.0 for Wndows® (© 1997 by SPSS Inc.). The inhibition constants (Ki) were calculated using the Cheng Prusoff equation (Ki = IC50/(1+(L/KD)), where L = concentration of radioligand in the assay, and KD = affinity of the radioligand for the receptor). A scatchard plot is used to determine the Kd.

### Human prostanoid EP₂ receptor cellular functional assay

### Protocol

Human CHO cells expressing the recombinant human prostanoid EP₂ receptor were suspended in assay medium (HBSS buffer (Invitrogen) containing 20 mM HEPES (pH7.4) and 500 µM isobutyl-methylxanthine IBMX) and plated out to yield approximately 1x104 cells/well in a 96-well. Following this, cells were incubated with agonists for 30 min in the presence of the test compounds. For stimulated control measurements, separate assay wells contain 10 µM PGE₂ (control specific agonist). All incubations were carried out at 37 °C in a 5% CO₂ atmosphere. Following incubation, the amount of cAMP in each well was determined by HTRF method. The cells are lysed and the fluorescence acceptor (D2-labeled cAMP) and fluorescence donor (anti-cAMP antibody labeled with europium cryptate) are added. After 60 min at room temperature, the fluorescence transfer is measured at ⊐ex=337 nm and ⊐em=620 and 665 nm using a microplate reader (Rubystar, BMG).

The cAMP concentration is determined by dividing the signal measured at 665 nm by that measured at 620 nm (ratio).

The standard reference agonist PGE₂ was tested at several concentrations to generate a concentration-response curve to determined EC_{50.}

### Analysis and expression of results

The results are expressed as a percent of control specific agonist response ((measured specific response/control specific agonist response) x 100) obtained in the presence of the test compounds. The EC50 values (concentration producing a half-maximal specific response) were determined by non-linear regression analysis of the concentration-response curves generated with mean replicate values using Hill equation curve fitting (Y = D + [(A - D)/(1 + (C/C50)nH)], where Y = specific response, D = minimum specific response, A = maximum specific response, C = compound concentration, and C50 = EC50 and nH = slope factor). This analysis was performed using a software developed at Cerep (Hill software) and validated by comparison with data generated by the commercial software SigmaPlot® 4.0 for Wndows® (© 1997 by SPSS Inc.).

### Rabbit corneal permeability using Ussing chamber

Ussing chambers were used for the permeation study each day of experiment.
3 ml of solution was placed in the donor side of Ussing chambers and 3ml of Ringer solution in the receiver side. Freshly removed rabbit corneal tissue was placed between the two chambers.

Temperature was maintained at 37°C during all the flux studies and oxygenation was provided by a continuous perfusion of carbogen (oxygen/carbonic acid) (95/5),

Rabbits were euthanized and the 6 corneas were removed and used immediately. 500 µL of receptor side liquid were removed from Ussing chambers and replaced by fresh buffer. The samples were analyzed immediately.

100µL of donor side liquid were removed from Ussing chambers and not replaced. The samples were diluted immediately after collection and analyzed after dilution. Analysis was performed on HPLC (Stationary phase: C18

Particule size: 3 µm Length: 5 cm) using a gradient from 5 to 95% ACN/water (0.1 % formic acid)

At the end of the sampling period, all corneas were discarded.

### Rabbit corneal homogenate hydrolysis rate

### Enzyme solution preparation

4 rabbits were euthanazied and the 8 corneas were immediately removed.

The corneas were scratched and placed in a tube with 50mL of Ringer solution at 4°C.

After homogenization, the tube was centrifuged 10 min and the supernatant was used immediately.

### Solution preparation

Six solutions were prepared: two at 10 µg/mL, two at 1 µg/mL and two at 0.1 µg/ml for each prodrug.

The tubes containing the solution at 10; 1 and 0.1 µg/mL in Ringer solution were placed in a water bath at 37°C and 200µL and aliquots were taken at the following sampling times: To, 0.5 h, 1h, 2h and 4h.

The concentration of remaining prodrug and formed drug were assayed using LCMSMS method.

Concentrations were obtained at T0, T0.5h, T1 h, T2h and T4h in the metabolism solution of prodrugs at 0.1 µg/mL, 1µg/mL and 10 µg/mL. Prodrug and drug were measured.

| Compound | In vitro functional assay EP2 (h) EC50 (nM) | % response | In vitro potency EP4 (h) IC50 (nM) | Overall Rabbit corneal permeability Papp (cm-6/s) | Rabbit corneal homogenate hydrolysis rate t1/2 (min) |
|---|---|---|---|---|---|
| CP-544326* | 46 | 72 | NC | | |
| PF-04217329° | NA | NA | NA | 19 | <4 |
| N° 1 | 110 | 74 | NC | | |
| N° 2 | 28 | 82 | NC | | |
| N° 5 | NA | NA | NA | 0.17 | 90 |
| N° 6 | 69 | 66% | NC | | |
| N° 7 | 160 | 61% | NC | | |
| N° 8 | 160 | 65% | NC | | |
| N° 9 | 55 | 80% | NC | | |
| N° 10 | 35 | 74% | NC | | |
| N° 11 | 13 | 88% | NC | | |
| N° 12 | 99 | 73% | NC | | |
| N° 13 | 26 | 80% | NC | | |
| N° 14 | 73 | 77% | > 300 | | |
| N° 15 | 24 | 76% | NC | | |
| N° 16 | 10 | 89% | NC | | |
| N° 17 | 52 | 86% | NC | | |
| N° 18 | 34 | 74% | NC | | |
| N° 19 | 17 | 70% | NC | | |
| N° 20 | 18 | 70% | NC | | |
| N° 21 | 45 | 78% | > 300 | | |

| | | | | | |
|---|---|---|---|---|---|
| *: corresponds to the drug of taprenepag °: corresponds to the taprenepag. | | | | | |

Taprenepag is a prodrug which was until recently in Phase II for the treatment of glaucoma.
NC: non calculable (inactive up to 300 nM)
NA: not applicable

### In vivo activity of compounds according to the invention:

Ten adult female cynomolgus monkeys, each weighing 3-5 kg, in which glaucoma had been induced unilaterally by diode laser photocoagulation of the mid-trabecular meshwork, were used in this study.

Intraocular pressure (IOP) was measured at 0 hr (before drug administration) and then hourly until 6 hrs after drug administration on one baseline day, one vehicle-treatment day, and treatment days 1, 3, and 5 with 0.01% of compound N° 15.

Compound N° 15 was tested in two groups of 4 monkeys for a total of 8 monkeys.

Following one baseline day (untreated) and one vehicle-treatment day (50µl (25µl x 2), drop of vehicle to the glaucomatous eye at 9:30 (a 50µl drop (25µl x 2) of compound N° 15 were topically applied to the glaucomatous eye *only* once daily at 9:30 am for *5 consecutive days.*

Slit-lamp examination for the detection of aqueous humor flare and cells was performed in a dark room before drug administration and at 1, 3 and 5 hrs after dosing on baseline day and at the same times on treatment days 1, 3 and 5.

Two-tailed paired and unpaired t test and the ANOVA test were used for statistical analysis.

Results are presented in Fig. 1, and show that the overall IOP is reduced by 22% after a daily administration over 5 days.

It is therefore apparent that the compounds of the invention have a full agonist effect on EP2 receptors and are selective over EP4 receptors and their prodrugs have a permeability through the cornea and are hydrolyzed by the estereases of the cornea. The compounds have a slow hydrolysis rate in the rabbit cornea and a suitable permeability, which correlates with a more sustained duration in vivo, compared to competitor.

The compounds according to the invention can therefore be used for preparing medicaments, especially medicaments which are inhibitors of EP2 receptors.

Accordingly, in another of its aspects, the invention provides medicaments which comprise a compound of formula (I), or one of its prodrugs.

In another of its aspects, the invention provides medicaments which comprise a prodrug of formula (II).

These medicaments are employed therapeutically, especially in the treatment and/or the prevention of glaucoma.

According to another of its aspects, the present invention relates to pharmaceutical compositions comprising as active principle a compound according to the invention. In an aspect, the compound according to the invention is a compound of formula (I) or one of is prodrugs. In another aspect, the compounds according to the invention are compounds of formula II. These pharmaceutical compositions comprise an effective dose of at least one compound according to the invention, or one of its prodrugs, and also at least one pharmaceutically acceptable excipient.

The said excipients are selected, in accordance with the pharmaceutical form and method of administration desired, from the customary excipients, which are known to a person skilled in the art.

In the pharmaceutical compositions of the present invention for oral, sublingual, subcutaneous, intramuscular, intravenous, topical, local, intra-tracheal, intranasal, transdermal, intraocular or rectal administration, the active principle of formula (I) above, or one of its prodrugs, may be administered in a unit administration form, in a mixture with conventional pharmaceutical excipients, to animals and to human beings for the prophylaxis and/or treatment of the above disorders or diseases.

The unit administration forms appropriate include oral forms such as tablets, soft or hard gel capsules, powders, granules and oral solutions or suspensions, sublingual, buccal, intratracheal, intra-ocular and intranasal administration forms, forms for inhalative, topical, transdermal, subcutaneous, intra-muscular or intravenous administration, rectal administration forms and implants. For topical application it is possible to use the compounds or their prodrugs according to the invention in creams, gels, ointments, eye drops or lotions.

As an example, a unit administration form of a compound according to the invention in eye drop form may comprise the following components:

| | |
|---|---|
| Compound according to the invention: | 1% w/v |
| Dimethyl sufoxide + PVP K-30: | 1% w/v |
| Benzalkonium chloride: | 0.01-0.02 % w/v |
| Ededate disodium: | 0.005-0.1% w/v |
| Boric acid: | quantity sufficient (QS) to buffer capacity |
| Sodium borate: | NMT 0.5% |
| Sodium chloride: | QS to osmolality 308 mOsm/kg |
| Polysorbate 80 | 0.1-3% |
| HPMC 4000 cps | 0.45-1% |
| HCl/NaOH | QS to pH 5-6 |
| Purified water | QS to required volume |

The present invention, according to another of its aspects, also provides a method of treating the pathologies indicated above, which comprises administering to a patient an effective dose of a compound according to the invention, or one of its prodrugs.

## Claims

1. Compound of the formula (I) 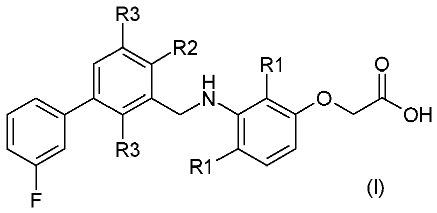 or one of its prodrugs, in which:
R1 represents, independently from each other, H, a (C₁-C₆) alkyl or an halogen,
R2 represents H, an halogen, or a (C₁-C₆)alkyl,
R3 represents, independently from each other, H, an halogen, -OH, -O-(C₁-C₆) alkyl or a (C₁-C₆) alkyl.
with the provision that the compounds of formula (I) are not:
Molecule A:
2-[2,4-difluoro-3-[[2-fluoro-3-(3-fluorophenyl)phenyl]methylamino]phenoxy]acetic acid Molecule B:
2-[2,4-difluoro-3-[[3-(3-fluorophenyl)-2-methyl-phenyl]methylamino]phenoxy]acetic acid
Molecule C:
2-[2,4-difluoro-3-[[3-(3-fluorophenyl)-2-hydroxy-phenyl]methylamino]phenoxy]acetic acid
Molecule D: 2-[2-fluoro-3-[[2-fluoro-5-(3-fluorophenyl)phenyl]methylamino]phenoxy]acetic acid, and
Molecule E: 2-[4-fluoro-3-[[2-fluoro-5-(3-fluorophenyl)phenyl]methylamino]phenoxy]acetic acid.

2. Compound of formula (I) according to Claim 1 wherein R1 represents, independently from each other, a (C₁-C₆) alkyl or an halogen.

3. Compound of formula (I) according to Claim 1 or claim 2, wherein the prodrug is an ester of the carboxylic moiety of compound (I).

4. Compound of formula (I) according to one of claims 1 to 3, wherein the prodrug is the carboxylic acid moiety of formula (I) protected by a (C₁-C₆)alkyl, an ethyl-morpholine, -O-CH(CH₃)-OCOCH₃, -CH₂-CH₂-OH, -CH-(CH₂OH)₂ -CH₂-CH-(CH₂OH)₂ or -CH₂-C-(CH₂OH)₃.

5. Compound of formula (I) according to one of claims 1 to 4, wherein the prodrug of the compound of formula (I) is a compound of formula (II): 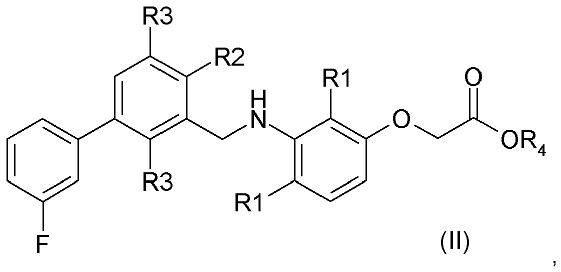 in which R1, R2 and R3 are as mentioned above, and R4 is (C₁-C₆) alkyl.

6. Compounds of formula (I) according to Claim 1 which are selected from the following list:
N° 1: 2-[3-[[2-fluoro-5-(3-fluorophenyl)phenyl]methylamino]phenoxy]acetic acid N° 2: 2-[2,4-difluoro-3-[[2-fluoro-5-(3-fluorophenyl)phenyl]methylamino]phenoxy] acetic acid
N° 6: 2-[2,4-difluoro-3-[[5-(3-fluorophenyl)-2-methyl-phenyl]methylamino]phenoxy] acetic acid
N° 7: 2-[2,4-difluoro-3-[[2-fluoro-3-(3-fluorophenyl)-5-hydroxy-phenyl]methylamino] phenoxy]acetic acid
N° 8: 2-[2,4-difluoro-3-[[3-(3-fluorophenyl)phenyl]methylamino]phenoxy]acetic acid N° 9: 2-[2,4-difluoro-3-[[2-fluoro-3-(3-fluorophenyl)-5-methyl-phenyl]methylamino] phenoxy] acetic acid
N° 10: 2-[2,4-difluoro-3-[[3-(3-fluorophenyl)-5-methyl-phenyl]methylamino]phenoxy] acetic acid
N° 11: 2-[2,4-difluoro-3-[[2-fluoro-5-(3-fluorophenyl)-3-methyl-phenyl]methylamino] phenoxy]acetic acid
N° 12: 2-[2-fluoro-3-[[2-fluoro-5-(3-fluorophenyl)phenyl]methylamino]-4-methyl-phenoxy] acetic acid
N° 13: 2-[3-[[2-fluoro-5-(3-fluorophenyl)phenyl]methylamino]-2,4-dimethyl-phenoxy]acetic acid
N° 14: 2-[4-chloro-2-fluoro-3-[[2-fluoro-5-(3-fluorophenyl)phenyl]methylamino]phenoxy] acetic acid
N° 15: 2-[3-[[2-chloro-5-(3-fluorophenyl)phenyl]methylamino]-2,4-difluoro-phenoxy]acetic acid
N° 16: 2-[4-fluoro-3-[[2-fluoro-5-(3-fluorophenyl)phenyl]methylamino]-2-methyl-phenoxy] acetic acid
N° 17: 2-[2,4-difluoro-3-[[3-fluoro-5-(3-fluorophenyl)phenyl]methylamino]phenoxy]acetic acid
N° 18: 2-[2-chloro-4-fluoro-3-[[2-fluoro-5-(3-fluorophenyl)phenyl]methylamino]phenoxy] acetic acid
N° 19: 2-[2,4-difluoro-3-[[3-(3-fluorophenyl)-5-hydroxy-phenyl]methylamino]phenoxy] acetic acid
N° 20: 2-[2,4-difluoro-3-[[3-(3-fluorophenyl)-5-methoxy-phenyl]methylamino]phenoxy] acetic acid
N° 21: 2-[2,4-dichloro-3-[[2-fluoro-5-(3-fluorophenyl)phenyl]methylamino]phenoxy]acetic acid

7. Prodrug of compounds of formula (I) according to one of claims 1 to 6 which are selected from the following list:
N° 3: methyl 2-[2,4-difluoro-3-[[2-fluoro-5-(3-fluorophenyl)phenyl]methylamino] phenoxy] acetate
N° 4: ethyl 2-[2,4-difluoro-3-[[2-fluoro-5-(3-fluorophenyl)phenyl]methylamino] phenoxy] acetate
N° 5: isopropyl 2-[2,4-difluoro-3-[[2-fluoro-5-(3-fluorophenyl)phenyl]methylamino] phenoxy]acetate

8. Medicament, **characterized in that** it comprises a compound of formula (I) or one of its prodrugs according to any one of Claims 1 to 7.

9. Pharmaceutical composition, **characterized in that** it comprises a compound of formula (I) or one of its prodrugs according to any one of Claims 1 to 7, and also at least one pharmaceutically acceptable excipient.

10. Use of a compound of formula (I) or one of its prodrugs according to any one of Claims 1 to 7 for preparing a medicament intended for the treatment and/or the prevention of a disease involving an agonist effect on EP2 receptor.

11. Use according to Claim 10 for preparing a medicament intended for the treatment and/or the prevention of glaucoma.

12. A method of treatment of glaucoma, which comprises administering to a patient an effective dose of a compound of formula (I) or one of its prodrugs, according to any one of Claims 1 to 7.

13. Compound of formula (I) or one of its prodrugs according to any one of Claims 1 to 7 as a medicament.
